# EUROPÄISCHE PATENTANMELDUNG

(11) **EP 4 663 649 A1**
(43) Veröffentlichungstag der Anmeldung: **17.12.2025**
(21) Anmeldenummer: 24182190.9
(22) Anmeldetag: 14.06.2024
(51) Int. Cl.: C07F 9/6571

(54) **BISPHOSPHITE MIT EINEM 3,4-DIALKYLPHENOL-FLÜGELBAUSTEIN**

(71) Anmelder: Evonik Oxeno GmbH & Co. KG, 45772 Marl (DE)
(72) Erfinder: BÜKER, Julia, 44787 Bochum (DE); FRANKE, Robert, 45772 Marl (DE); FRIDAG, Dirk, 45721 Haltern am See (DE); BILKE, Marius, 45711 Datteln (DE); BRUNS, Bastian, 44791 Bochum (DE); BÜRK, Vincent, 45665 Recklinghausen (DE); KUCMIERCZYK, Peter, 44628 Herne (DE); MARKOVIC, Ana, 45721 Haltern am See (DE); PESCHKE, Roland, 47057 Duisburg (DE); WESSNER, Maximilian, 44139 Dortmund (DE); SALE, Anna Chiara, 40474 Düsseldorf (DE)
(74) Vertreter: Evonik Patent Association

(57) **Zusammenfassung**

Bisphosphite mit einem 3,4-Dialkylphenol-Flügelbaustein und deren Verwendung in der Hydroformylierung.

## Beschreibung

Die Erfindung betrifft Bisphosphite mit einem 3,4-Dialkylphenol-Flügelbaustein und deren Verwendung in der Hydroformylierung.

Phosphorhaltige Verbindungen spielen als Liganden in einer Vielzahl von Reaktionen eine entscheidende Rolle, z.B. in der Hydrierung, in der Hydrocyanierung und auch in der Hydroformylierung.

In WO 02/00670 A1 werden Bisphosphitverbindungen, deren Matallkomplexe und Verwendung der Verbindungen und Komplexe in der Olefinhydroformylierung beschrieben. Unter anderem wird hier die Verbindung **(Ila)** gezeigt:

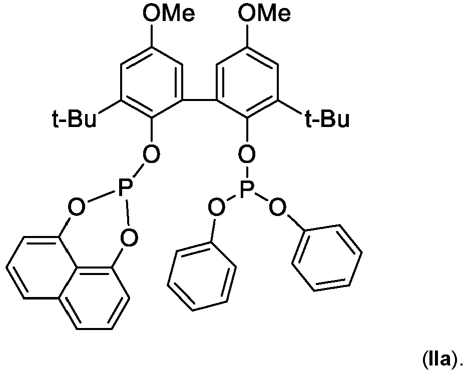

Die technische Aufgabe der Erfindung ist die Bereitstellung einer Verbindung, mit welcher bei der Hydroformylierung von Olefinen eine gute Ausbeute erzielt werden kann.

Die Aufgabe wird gelöst durch eine Verbindung gemäß Anspruch 1.

Verbindung gemäß Formel **(I):** 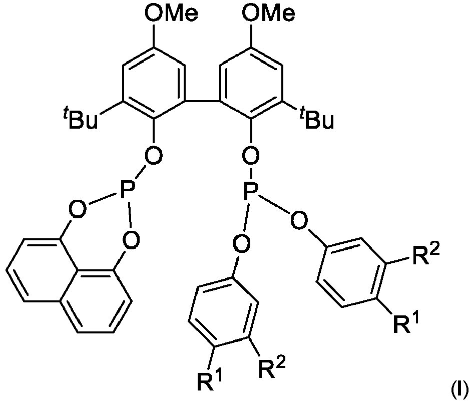 wobei R¹, R² für -(C₁-C₄)-Alkyl stehen.

In einer Ausführungsform stehen R¹, R² für -CH₃ oder ^{-*t*}Bu.

In einer Ausführungsform stehen R¹, R² für -CH₃.

In einer Ausführungsform weist die Verbindung die Struktur (1) auf:

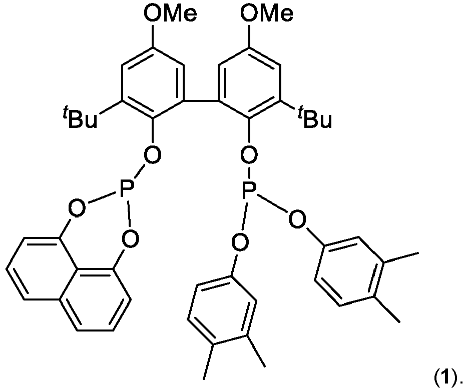

Neben den Verbindungen selbst wird auch ein Verfahren beansprucht, in dem die zuvor beschriebenen Verbindungen zum Einsatz kommen.

Verfahren umfassend die Verfahrensschritte:
a) Vorlegen eines Olefins;
b) Zugabe einer zuvor beschriebenen Verbindung;
c) Zugabe einer Substanz, welche Rh umfasst;
d) Zuführen von H₂ und CO;
e) Erwärmen des Reaktionsgemisches aus a) bis d), wobei das Olefin zu einem Aldehyd umgesetzt wird.

In einer Variante des Verfahrens ist die Substanz, welche Rh umfasst, ausgewählt aus: Rh(acac)(CO)₂, Rh(acac)(cod) (Umicore, acac = Acetylacetonat-Anion; cod = 1 ,5-Cyclooctadien), Rh₄CO₁₂.

In einer Variante des Verfahrens ist die Substanz, welche Rh umfasst, Rh(acac)(CO)₂.

Im Folgenden soll die Erfindung anhand von Ausführungsbeispielen näher erläutert werden.

### Synthese

### Erste Stufe

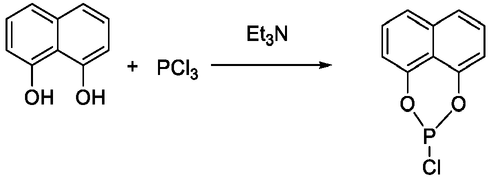

Es wurden 0,076 mol Naphthalin-1,8-diol über Nacht bei 50 °C mittels Ölpumpenvakuum getrocknet. Am darauffolgenden Tag wurde der Schlenk mit Argon geflutet und das Naphthalin-1,8-diol in 350 ml getrocknetem Toluol gelöst. In einem sekurierten Schlenk wurden 0,114 mol Phosphortrichlorid in 120 ml getrocknetem Toluol gelöst. Anschließend wurde die Naphthalin-1,8-diol-Lösung bei -20 °C langsam und stetig zur PCI₃-Lösung getropft. Danach wurden 0,165 mol Triethylamin langsam unter hoher Rührgeschwindigkeit bei -20 °C zu der Lösung hinzugetropft. Die Lösung wurde auf Raumtemperatur gebracht und über Nacht weiter gerührt. Am nächsten Tag wurde die Reaktionsmischung abgefrittet, der Filterkuchen mit zweimal je 25 ml Toluol gewaschen und das Filtrat mittels Ölpumpenvakuum bei 40 °C eingeengt.

### Ausbeute: 86%

### Zweite Stufe

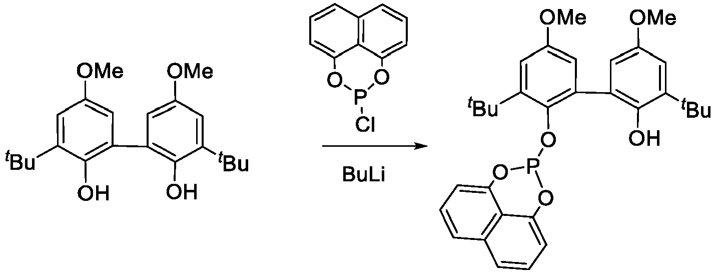

Es wurden 0,064 mol Biphenol über Nacht mittels Ölpumpenvakuum getrocknet. Am nächsten Morgen wurde der Schlenk mit Argon geflutet und das Biphenol in 200 ml getrocknetem THF gelöst. Anschließend wurde die Lösung auf -20 °C abgekühlt und tropfenweise 0,064 mol einer 1.6M n-Butyllithium-Lösung in Hexan hinzugegeben. Nach beendeter Zugabe wurde die Reaktionslösung noch 30 min bei -20 °C gerührt und dann langsam auf Raumtemperatur erwärmt. Unter Inertgasatmosphäre wurden 0,065 mol des Chlorophosphits in 100 ml getrocknetem THF gelöst. Danach wurde die Butyllithium-THF-Lösung langsam und stetig bei Raumtemperatur zur Biphenol-THF-Lösung gegeben. Die Reaktionslösung wurde über Nacht bei Raumtemperatur gerührt und am nächsten Morgen mittels Ölpumpenvakuum bei 40 °C bis zur Trockene eingeengt.

Zur Aufreinigung wurden zu dem erhaltenen Feststoff 700 mL entgastes n-Heptan gegeben, auf 70 °C erwärmt und für 2 Stunden rühren gelassen. Die heiße Lösung wurde über Celite abgefrittet, um die ausgefällten Salze abzutrennen. Die Lösung wurde im Anschluss auf 0 °C abgekühlt und für vier Stunden gerührt. Anschließend wurde der erhaltene Feststoff abgefrittet, zweimal mit je 50 mL kaltem Heptan gewaschen und mittels Ölpumpenvakuum getrocknet.

### Ausbeute: 66%

### Dritte Stufe

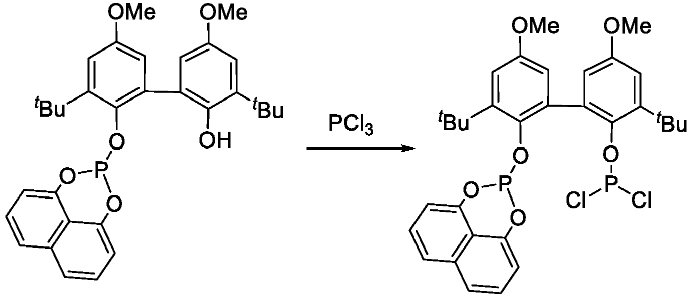

Unter Inertgasatmosphäre wurden 0,038 mol Organophosphit in 200 ml getrocknetem Toluol und 13,5 ml (0,095 mol, 2,5 eq.) Triethylamin gelöst. In einem sekurierten Schlenk wurden 0,046 mol Phosphortrichlorid in 100 ml getrocknetem Toluol gelöst und auf 0 °C abgekühlt. Im Anschluss wurde die Organophosphit-EtsN-Lösung langsam und stetig bei 0 °C zum PCl₃ zugetropft. Die Reaktionslösung wurde über Nacht bei Raumtemperatur gerührt. Das entstandene Ammoniumhydrochlorid wurde abgefrittet und mit zweimal je 50 ml getrocknetem Toluol gewaschen. Das erhaltene Filtrat wurde anschließend mittels Ölpumpenvakuum bei 40 °C bis zur Trockene eingeengt.

### Ausbeute: 91%

### Synthese (1)

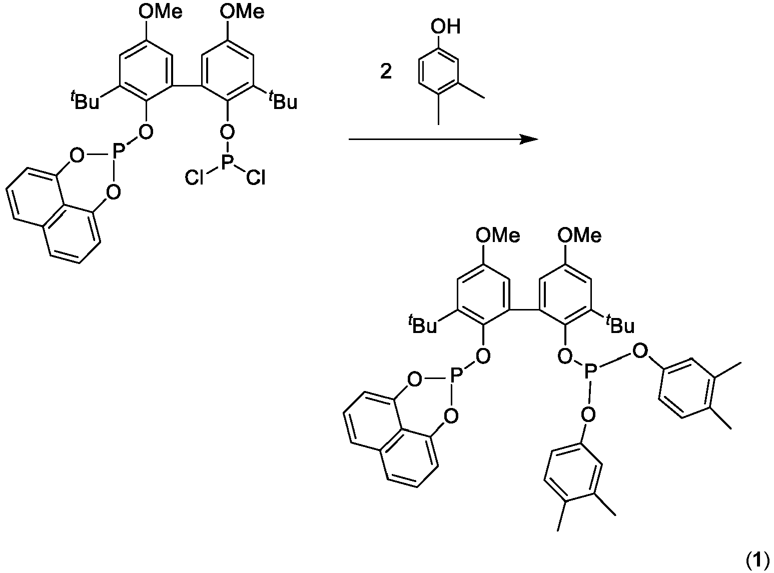

Unter Inertgasatmosphäre wurden 2,6 mmol Organodichlorophosphit abgewogen und in 30 ml getrocknetem Toluol suspendiert. Es wurden 6,6 mmol des Phenol-Derivats in einem Schlenk abgewogen und kurz mittels Ölpumpenvakuum sekuriert. Anschließend wurde der Schlenk mit Argon geflutet und das Phenol-Derivat in 20 ml getrocknetem Toluol gelöst und 13,1 mmol entgastes Triethylamin hinzugegeben. Anschließend wurde die Phenol-Lösung langsam und stetig bei Raumtemperatur zur Chlorophosphit-Suspension gegeben. Die Reaktionslösung wurde über Nacht bei Raumtemperatur gerührt. Das entstandene Ammoniumhydrochlorid wurde abgefrittet und zweimal mit je 10 ml getrocknetem Toluol nachgewaschen. Das erhaltene Filtrat wurde anschließend mittels Ölpumpenvakuum bei 40 °C bis zur Trockene eingeengt. Das getrocknete Filtrat wurde mittels Säulenchromatographie aufgereinigt.

### Ausbeute: 68%

Analog zur Verbindung **(1)** wurden die Verbindungen **(2)** und **(IIa)** hergestellt.

Bei den Verbindungen **(2)** und **(IIa)** handelt es sich um nichterfindungsgemäße Vergleichsverbindungen.

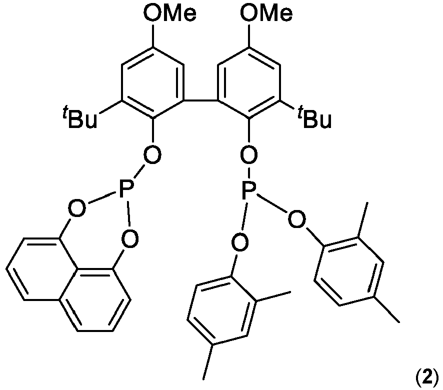

### Katalyseversuche

Es wird unter Argon-Atmosphäre gearbeitet. Reaktionsgefäße sind zuvor unter Einwirkung von Temperatur (80 °C) und Ölpumpenvakuum getrocknet worden. Flüssige Substanzen wurden für mind. 15 Minuten durch Einperlen von Argon entgast. Die Hydroformylierung wurde in einem mit Druckkonstanthaltung ausgestatteten 0,5 L-Autoklaven der Firma Berghof Products + Instruments GmbH, durchgeführt. Beheizt wird der Reaktor mittels Ölbad der Firma IKA. Der Reaktor dient zum Gasaustausch und als Temperierwerk. Innerhalb dieses Reaktors wurden 5 Glasvials (20mL), gefüllt mit Katalysatorlösung und Magnetrührfischen unter Argon gebördelt, so platziert, dass ein Gasaustausch zwischen Vials und Reaktorraum möglich ist. Durch im Reaktor enthaltenes Thermalöl wurden die Glasvials temperiert. Angegebene Reaktionstemperaturen wurden im inneren der Glasvials gemessen. Als Substrat eingesetzt wurde n-Octen (Oxeno GmbH, Octenisomerengemisch aus 1-Octen: 3 %; cis+trans-2-Octen: 49 %; cis+trans-3-Octen: 29 %; cis+trans-4-Octen: 16 %; gerüstisomere Octene: 3 %).

Für einen Versuchslauf wurde eine Stammlösung vorab unter Argonatmosphäre vorbereitet. Hierfür wurden 0,0127 g Rh(acac)(CO)₂ und die entsprechende Menge an Phosphitverbindung (MV Lig:Rh = 5:1) eingewogen und mit 48,0 ml Toluol aufgefüllt. Von dieser Lösung wurden je ca. 8 mL auf die Vials verteilt und die genaue Menge gewogen. Die Vials wurden im Reaktor platziert und dieser verschlossen. Es wurde dreimal mit Argon und dreimal mit Synthesegas (Linde; H₂ (99,999 %) : CO (99,997%) = 1:1) gespült. Nach erfolgter Druckprüfung wurde der Autoklav bei einem Gesamtdruck von 10 bar unter Rühren (900 U/min) auf die angestrebte Temperatur von 120°C aufgeheizt. Nach Erreichen der Reaktionstemperatur wurde der Synthesegasdruck auf 20 bar erhöht und das Substrat mittels HPLC-Pumpe mit je 2 mL zum Reaktionsstart zudosiert. Daraus ergibt sich eine Rh-Konzentration von 100 ppm. Nach 1 h Reaktionsstart bei konstantem Druck wurde eine Probe je Vial gezogen und unverdünnt gaschromatographisch analysiert: HP 6890, Petrocol^{®} DH 150, 150 m x 0,25 mm x 1 µm. Die quantitative Bestimmung von Restolefin und Aldehyd erfolgte gegen das Lösungsmittel Toluol als interner Standard. Die in der untenstehenden Tabelle gelisteten Ergebnisse stellen den Mittelwert über einen Versuchslauf da.

### Ergebnisse der Katalyseversuche

### [Rh]: 100 ppm, p: 20 bar, T: 120 °C; t: 1 h

Das eingesetzte n-Octen-Gemisch bestand aus den C8-Isomeren: 1-Octen, cis-2-Octen, trans-2-Octen, cis-3-Octen, trans-3-Octen, cis-4-Octen und trans-4-Octen.

**Tabelle 1:**

| Ligand | Ausbeute [%] |
|---|---|
| **(1)*** | 40 |
| **(2)** | 35 |
| **(IIa)** | 39 |

| | |
|---|---|
| * erfindungsgemäßes Ausführungsbeispiel | |

Die durchgeführten Versuche belegen, dass die gestellte Aufgabe durch eine erfindungsgemäße Verbindung gelöst wird.

## Patentansprüche

1. Verbindung gemäß Formel **(I):** 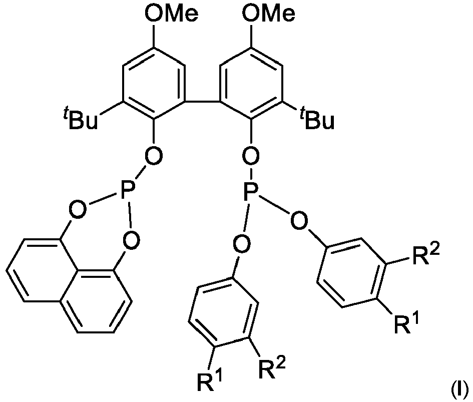 wobei R¹, R² für -(C₁-C₄)-Alkyl stehen.

2. Verbindung nach Anspruch 1,
wobei R¹, R² für -CH₃ oder -*^{t}*Bu stehen.

3. Verbindung nach einem der Ansprüche 1 oder 2,
wobei R¹, R² für -CH₃ stehen.

4. Verbindung nach einem der Ansprüche 1 bis 3,
wobei die Verbindung die Struktur (1) aufweist:

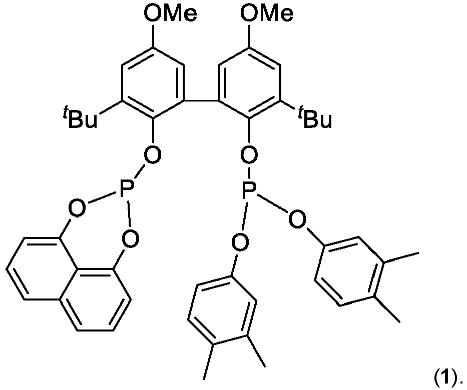

5. Verfahren umfassend die Verfahrensschritte:
a) Vorlegen eines Olefins;
b) Zugabe einer Verbindung nach einem der Ansprüche 1 bis 4;
c) Zugabe einer Substanz, welche Rh umfasst;
d) Zuführen von H₂ und CO;
e) Erwärmen des Reaktionsgemisches aus a) bis d), wobei das Olefin zu einem Aldehyd umgesetzt wird.

6. Verfahren nach Anspruch 5,
wobei die Substanz, welche Rh umfasst, ausgewählt ist aus: Rh(acac)(CO)₂, Rh(acac)(cod) (Umicore, acac = Acetylacetonat-Anion; cod = 1 ,5-Cyclooctadien), Rh₄CO₁₂.

7. Verfahren nach einem der Ansprüche 5 oder 6,
wobei die Substanz, welche Rh umfasst, Rh(acac)(CO)₂ ist.
